# EUROPEAN PATENT SPECIFICATION

(11) **EP 1 682 867 B1**
(45) Date of publication and mention of the grant of the patent: **07.05.2008**
(21) Application number: 04799294.6
(22) Date of filing: 08.11.2004
(51) Int. Cl.: G01N 1/28, C12M 3/08, B02C 19/08

(54) **SAMPLE HOMOGENISER**
PROBENHOMOGENISATOR
HOMOGENEISATEUR D'ECHANTILLONS

(30) Priority: 14.11.2003 IE 20030856
(43) Date of publication of application: 26.07.2006
(73) Proprietor: ENFER TECHNOLOGY LIMITED, Dublin 2 (IE)
(72) Inventor: CLARKE, John, Ballinasloe, CountyGalway (IE); LE FEUVRE, Peter, Castel, Guernsey GY5 7XA (GB); INDER, David, Vale, Guernsey GY3 5QX (GB)
(74) Representative: Gates, Marie Christina Esther
(86) International application number: PCT/IE2004/000152
(87) International publication number: WO 2005/047866

(56) References cited:
- EP-A- 0 590 504
- WO-A-00/02031
- WO-A-02/48679
- WO-A-91/02590
- GB-A- 247 943
- GB-A- 2 100 137
- US-A- 4 505 433
- US-A- 4 715 545
- US-A- 5 390 859
- US-A- 5 636 921

## Description

### FIELD OF THE INVENTION

The present invention relates to equipment for de-constituting biological samples, in particular for the preparation of homogenised samples for-routine diagnostic or research testing.

### BACKGROUND TO THE INVENTION

Most existing homogenisation techniques of tissue do not readily lend themselves to automation or high throughput use. For example a stomacher instrument uses a plastic bag to contain the tissue sample and liquid. Automatically sampling such a bag is very difficult.

Many existing methods of homogenising biological tissue use ceramic, glass, plastic or metal homogenisers or containers, designed for repeated use. While sterilising or washing with hot water and detergent is sufficient to remove most bacterial, viral and nucleotide contamination, often, minute traces can remain. Furthermore, biological pathogens such as prions are reported to be difficult to render inactive by the above methods, requiring disposal of any materials, such as surgical instruments, that may have come in contact with them. In addition, accessing the resulting homogenate often requires at least partial disassembly of the apparatus, which can result in splashes or aerosols being formed, which provide a source of contamination and infection.

The present invention overcomes these disadvantages by providing an economically viable method of providing a disposable homogeniser that does not require disassembly after use in order to access the homogenate.

International Patent Application WO 02/48679 entitled a "Device for disintegrating biological samples" teaches a deconstituting device for the preparation of biological samples. The device comprises a container for holding the tissue to be deconstituted; a shaft (with a blade on the end) mounted for rotation inside the said container.

The shaft is supported axially by ball coupling means and has engagement means on its end outside the container for coupling the shaft to a motor. The device is envisaged to work as an automatic apparatus with a plurality of housings for receiving the container and a conveyor means for moving the containers.

As will be discussed bellow, the central shaft of the present invention can communicate the homogenised material, away from the chamber that homogenisation is carried out into another chamber where assay priming and/or the assay itself may be carried out Thus only the most thoroughly homogenised fraction of the sample is assayed. Furthermore, compared with the prior art the homogenised sample is transported from the apparatus more quickly and easily (parts do not need to be disengaged and less parts are involved) and thus more economically as the apparatus is more easily adapted to mechanisation.

United States Patent US 4,505,433 provides a device comprising a container and a grinder receivable in the interior of the container. The grinder has a grinding head having a concave shape closely conforming to the concave shape of the container interior bottom, a cap that is screwed onto the upper portion of the container, and a compressible intermediate and body section joining the grinding head and the grinder top. A reservoir containing bacterial maintenance fluid, such as a frangible glass ampoule containing such fluid is carried within the compressible intermediate section. In use, the cap is screwed on by hand, which forces the grinding head into the bottom of the container interior. Furthermore, the rotation places the glass ampoule under compression, thus breaking it and releasing the bacterial maintenance fluid so as to keep the tissue moist and reduce any molecular oxygen present. It is important to note that neither WO 02/48679 or US 4,505,433 teach a means for the removal of the homogenised tissue from the apparatus, in particular they do not teach or disclose a central shaft capable of communicating the homogenised material from the homogenisation chamber to an assay or assay priming area.

Therefore, for both veterinary and economic reasons, there exists a need to provide a means for consistently and routinely disrupting tissue so as to provide a sample suitable for the efficient, reliable, repeatable and accurate assay for disease The invention has been described with reference to the application to TSE related tissues, but the invention can suitably be used for homogenising any biological material, and providing a degree of uniformity, consistency and safety whether the sample is homogenised mechanically as one of a plurality of samples or singly, or is hand-homogenised singly or as a plurality of samples.

### OBJECT OF THE INVENTION

One object of the present invention is to provide an economical, reliable and user-friendly means for the automatic or manual deconstitution or homogenisation of biological tissue samples. An additional object of the invention is the provision of a means for communicating the homogenised material from the homogenisation chamber by a process that does not necessitate either the opening of the homogenisation machinery or the introduction of an external sample extraction means. It is further desirable that any such means for removing homogenised samples from the homogenisation chamber does not expose the contents of the chamber to contaminating particles.

An additional object of the invention is to provide a means that ensures that any homogenisation performed is any reproducible for all samples submitted for testing, while simultaneously providing an excellent level of homogenisation.

### SUMMARY OF THE INVENTION

According to the present invention there is provided comminuter for homogenising or comminuting (the terms are used synonymously throughout this document) a sample of material, preferably a sample of biological tissue, most preferably a sample of central nervous tissue.

The homogeniser comprises a grinder, comprising
(i) a hollow shaft defining an interior channel,
(ii) a grinding head and
(iii) at least one port;
characterized in that the grinding head defines a closed end of the shaft and the at least one port is located on the elongate sides of the shaft, said port being dimensioned so that only comminuted material may flow into the interior channel of the shaft.

The homogeniser may also comprise: a container, and a grinder, wherein
the container comprises an interior defined by sidewalls, an upper portion having an upwardly open top communicable with an exterior of the container, and an interior bottom, and
the grinder comprises
(i) a hollow shaft locatable within the container, the hollow shaft defining an interior channel,
(ii) a grinding head provided at one end of the shaft, the grinding head defining a closed and of the shaft
(iii) at least one port located on the elongate sides of the shaft dimensioned such that only comminuted material may flow into the interior channel of the shaft.

In an alternative embodiment of the present invention the grinding head comprises at least one blade. In a preferred embodiment of the present invention the grinding head is shaped to compliment the shape of the interior bottom, preferably wherein the shape is selected from the group consisting of substantially flat, substantially conical, substantially frustroconical, substantially hemispherical and substantially spherical-cap shaped. Preferably, the interior bottom and the grinding head each comprise a sample-engaging surface, although in an alternative embodiment of the present invention at least one of the sample-engaging surfaces is substantially smooth.

In a preferred embodiment of the present invention at least one of the sample-engaging surfaces is an abrasive surface, preferably wherein the abrasive surface comprises at least one abrasive feature selected from the group consisting of protrusions, griddles, indentations, hatching or embedded particles. Preferably, the at least one abrasive surface is integrally formed with at least one of the grinding head or the interior bottom, although in an alternative embodiment of the present invention the at least one abrasive surface is independently formed with at least one of the grinding head or the interior bottom.

In the present invention the port is dimensioned so that only comminuted material may pass into the interior channel of the shaft from the container. This provides the advantage that non-homogenised material, or debris does not mix with the homogenate; preferably the port comprises a slit formed in the shaft and is positioned on the shaft proximate to the grinding head. Preferably, there is provided a plurality of ports on the shaft.

In an alternative embodiment of the present invention, there is provided a means to reversibly close the at least one port. This is not necessary in all embodiments, as the centrifugal force generated during rotation will prevent egress of the homogenate into the interior channel but closure of the ports may be desirable during and possibly after comminuting of the sample. Such an alternative embodiment is particularly desirable when the at least one port is positioned on the shaft such that when the apparatus is used in combination with a defined volume of a solution separable into at least two layers defined by differing density gradients, the positioning of the at least one port permits egress of at least one of the layers into the interior channel and permits the prevention of egress of at least one of the layers into the interior channel. This embodiment operates particularly in combination with the embodiment providing reversible closure of the ports. The user can homogenate the sample with the ports in the closed position, and allow the layers to settle. Following separation of the homogenate into discrete density layers the ports can be opened allowing egress of the desired layer or layers.

In a preferred embodiment of the present invention the upper portion of the shaft is provided with an engagement means, the engagement means providing a means of detachably engaging the shaft with a rotation device, or a rotation device adaptor, or a closure means. Preferably, the engagement means comprises at least one groove, projection or indentation on the upper portion of the shaft. It is further preferable that the engagement means is shaped to complement the shape of the rotation device, the rotation device adaptor, or the closure means. In a preferred embodiment, the engagement means comprises a pair of 180° helices in which the end of each helix most distal to the grinding head is separated from the end of the other helix by half of the perimeter of the shaft. Alternative arrangements are readily conceivable, such as cogs, teeth, screw and threads, holding pins and other commonly known means.

Preferably, the engagement of the engagement means with the rotation device, or the rotation device adaptor, or the closure means, permits movement of the rotation device, or the rotation device adaptor, or the closure means in at least one direction to be translated into movement of the shaft in the same at least one direction. Preferably, the direction is rotational about the central axis of the shaft.

In an alternative embodiment, the engagement means is further provided with a coupling means to provide reversible and secure coupling of the shaft to the rotation device or to the rotation device adaptor or to the closure means. Preferably, the coupling means comprises a grove, projection or indentation adapted to engage with the rotation device or with the rotation device adaptor or with the closure means such that movement of the rotation device, or the rotation device adaptor, or the closure means in one of at leat two directions is translated into movement of the shaft in the same direction. Preferably, the first of the at least two directions is rotation about the shaft axis and the second of the at least two directions is substantially parallel to the shaft axis. This embodiment permits the translation of both a rotational movement to the shaft about the central axis and a perpendicular "up-down" movement, to permit a jackhammer like action.

In an alternative embodiment, the closure means is adapted to be securely and reversibly engagable with the engagement means and provide a substantially watertight seal therein. Furthermore, the invention provides that the closure means further comprises a rotation device or a rotation device adaptor. This embodiment is particularly useful with a hand held and/or hand operated rotation device and is very suitable for low throughput use of the invention. The closure means therefore would. provide a means of attachment and means to transfer the directional force required to homogenize the material, as well as providing means to seal the interior channel.

In a preferred embodiment the shaft further comprises a biasing means for biasing the grinding head against the interior bottom of the container. Preferably, the grinder can nest within the container, such that the grinding head is proximate to the interior bottom, and a sample ofhomogenisable material can be contained between the grinding head and the interior bottom, and movement of the grinder relative to the container results in the sample of material being comminuted. Preferably, the grinder is moveable in at least one direction chosen from rotational movement and movement in a directions substantially parallel to the central axis.

Preferably, the homogeniser further comprises a cap, wherein the cap comprises a roof portion and an outer skirt engagable with the upper portion of the container and the roof portion further comprises an aperture adapted to permit the shaft of the grinder to protrude through the aperture. The cap may further comprise a shaft engaging means and a portion of the shaft is dimensioned to be engagable with the shaft engaging means. Preferably, the shaft engaging means extends from the periphery of the aperture. More preferably, the shaft engaging means comprises an internal continuous sidewall. In an alternative embodiment of the present invention, the shaft engaging means and/or the shaft is coated and/or comprises a reduced friction engaging means, such as a lubricant.

The cap can be provided with a securing means to reversibly secure the cap to the upper portion of the container, preferably wherein the securing means is selected from at least one of the group consisting of a push-fit mechanism, a snap-lock mechanism, thread and screw arrangement. In one embodiment of the present invention the snap-lock mechanism comprises a recess located on the outer skirt adapted to engage with a complimentary rim located on the sidewall of the container. Alternatively, the snap-lock mechanism comprises a rim located on the outer skirt adapted to engage with a complimentary recess located on the sidewall of the container.

The shaft may be provided with a restraining means to limit the movement of the shaft through the aperture. Such a restraining means can take the form of a collar located on the shaft This can have the advantage of limiting splashes of liquid from emerging through the aperture from the container. In alternative embodiment, the restraining means comprises a shoulder located on the shaft, wherein the shoulder is defined by a step-wise alteration in the radius of the shaft.

The internal continuous sidewall also advantageously limits the amount of lateral movement of the shaft. Furthermore, the height of the internal continuous sidewall relative to the roof of the cap and the respective distance between the grinding head and the restraining means can be varied by the designer or in an alternative embodiment to allow for a variation in the maximum amount of volume permitted between the interior bottom and grinding head when the apparatus is assembled. It will be appreciated that some of these advantages can be achieved by another means, such as a collar which can be positioned up and down the shaft, for example by a screw thread, also by a shaft engaging means which does not take the form of an internal continuous sidewall.

Preferably, at least one of the group consisting of the homogeniser, container, grinder or cap is substantially translucent. This permits the user to examine or visually inspect the sample material. It is also possible to construct the homogeniser such that the visual properties of the relevant portion do not interfere with any desired spectroscopic analysis. Preferably, the homogeniser is substantially composed of thermoplastics or metals and is preferably machined from solid or plastic casting or metal casting or infection moulded. Alternatively, the homogeniser is substantially composted of glass or ceramic.

In an alternative embodiment, at least one portion of the homogenised is composed of, impregnated with, or coated with a reactive material selected to react with a moiety intended for use within the homogeniser. For example the reactive material can be adapted to adhere to biological molecules that may be found in a homogenised sample. Also, the reactive material can be chosen from the group consisting of at least one antibody species, at least one enzyme species, at least one biological marker species.

There may be provided a means to aspirate the homogenate from the inner channel. Furthermore, there-may be provided a means to dispense material into the homogeniser. In an alternative embodiment, the present invention may further comprise a holding device.

Preferably, the container further comprises a positioning means engagable with the holding device, such that the positioning means permits the container to be engaged in a specific orientation relative to the holding device. Preferably, the positioning means is located distal to the upper portion. Preferably, the specific orientation permits a defined area of the container to be presented to an identification reader, such as a barcode scanner or reader of a global unique identifier. The user or designer may find it desirable to locate the identification reader on the holding device.

In an alternative embodiment, the homogeniser further comprises a rotation device. Preferably the rotation device further comprises a biasing means to provide a substantially constant force to the grinder when the rotation device is engaged with the engagement means, preferably in the form of a spring. The rotation device may be either mechanically or manually operable.

The rotation device may further comprise a means to aspirate the comminuted material from the interior space, and/or a means to dispense material into the homogeniser. Alternatively, either or both of these means can be provided on the holding device or at a separate location. The holding device and rotation device may be either independently or integrally formed.

Although the invention has been described as an homogeniser comprising a container, the invention also provides for an homogeniser comprising a grinder, wherein the grinder comprises
a hollow shaft connected to a grinding head, the shaft having an interior channel and the grinding head defining a closed end of the shaft, and
at least one port located on the elongate sides of the shaft and being dimensioned such that comminuted material may flow into the interior channel of the shaft.

It will be appreciated that this embodiment of the invention can be used effectively with pre-existing containers or alternative containers. It will also be appreciated that the features and embodiments as described herein relating to the homoginiser apply equally to the embodiment of the grinder alone and *vice versa.*

The invention also provides for a kit for homogenising a sample of material comprising a plurality of homogenisers substantially as described herein, or at least one homogenisers substantially as described herein and further comprising at least one of the group consisting of a rotation device, a rotation device adapter, a closure means, a holding device, a container, a cap, a grinder or biological or reactive coating.

The invention also provides for a method for homogenising a sample of material comprising the use of an homogeniser as herein described and
(i) placing the sample to be homogenised within the interior of the container such that it can be retained by the interior bottom,
(ii) inserting the shaft into the interior such that the grinding head is contactable with the sample,
(iii) placing the cap on the container such that the upper portion of the shaft extends through the aperture,
(iv) engaging the engagement means with the rotation device and optionally securably restraining the rotational movement of the container relative to the shaft optionally by means of the holding device.
(v) homogenising the sample material by means of movement of the shaft relative to the container,
(vi) removing homogenised material from the interior of the container into the interior channel of the shaft by means of at least one port.

In an alternative embodiment, the comminuted tissue in the interior channel of the shaft is removed from the comminuter by aspiration.

The invention also provides for use of an homogeniser, a kit for homogenising, or method of comminuting a sample as defined in the claims.

### BRIEF DESCRIPTION OF THE DRAWINGS

Figure 1 is a side-on view of one embodiment of a comminuter of the present invention.
Figure 2 is a cut away side view of an embodiment of the comminuter in accordance with the present invention, showing a grinder nested within a container with a cap.
Figure 3 shows a top perspective view of the comminuter with a grinder nested within a container with a cap.
Figure 4 shows a bottom perspective view of the comminuter with a grinder nested within a container within a cap.
Figure 5 is a top perspective cut away view of a comminuter of the present invention.
Figure 6 is a side view of a grinder of a preferred embodiment of the present invention.
Figure 7 is a top perspective cutaway view of a grinder.
Figure 8 is a bottom perspective view of a grinder.
Figure 9 a top perspective view of a grinder.
Figure 10 is a top perspective view of a container.
Figure 11 is a side-on cut away view of a container.
Figure 12 is a cutaway top perspective view of a container.
Figure 13 is a bottom view of the container.
Figure 14 is a top perspective view of a container.
Figure 15 is a bottom perspective view of a container.
Figure 16 is a cut away top perspective view of a cap.
Figure 17 is a cut away side view of a cap.
Figure 18 is a bottom perspective view of a cap.
Figure 19 is a bottom perspective view of a cap.
Figure 20 is a top perspective view of a cap.
Figure 21 shows a cut away side view of an alternative embodiment of the comminuter, showing a grinder nested within a container with a cap.
Figure 22 is a cut away side on view of an alternative embodiment of a grinder.
Figure 23 is a bottom perspective view of an alternative embodiment of a grinder.
Figure 24 is a top perspective view of an alternative embodiment of a grinder.
Figure 25 shows a side on view of a grinder, assembled with a container and a cap, engaging with a rotation means.
Figure 26 is a side on perspective view of an alternative embodiment of a grinder showing a coupling means.
Figure 27 is a perspective view of a closure means.

### DETAILED DESCRIPTION OF THE DRAWINGS

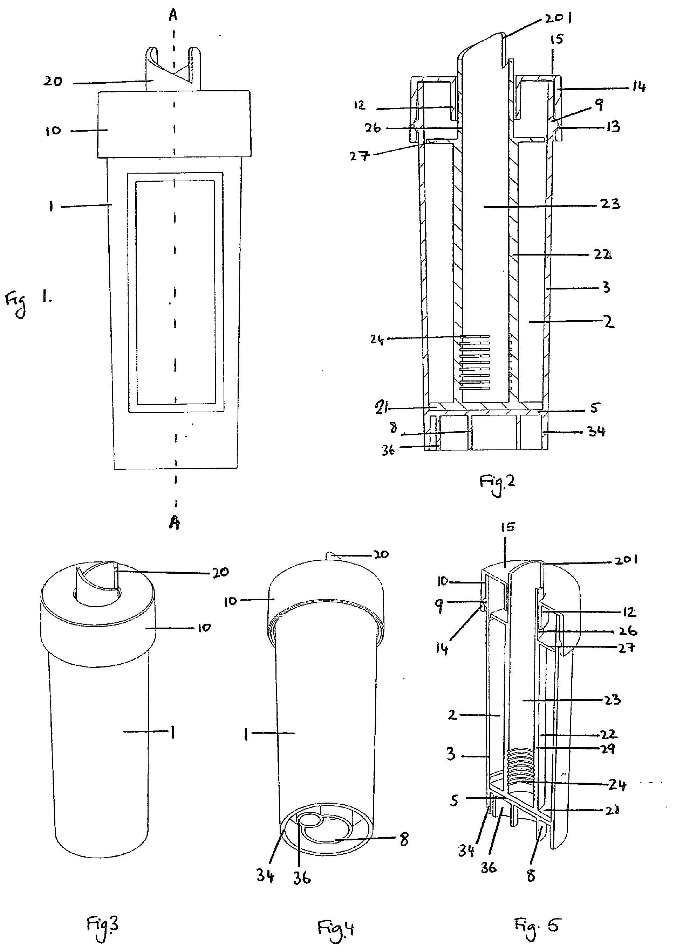

A preferred embodiment of the apparatus is depicted in Figures 1 to 5. Briefly, the apparatus comprises a grinder (20), nested within a container (1) adapted for receiving the grinder (20), and a cap (10). The container (1) is defined by an interior (2) defined by sidewalls (3), an upper portion (4) having an upwardly open top communicable with an exterior of the container, and an interior bottom (5).

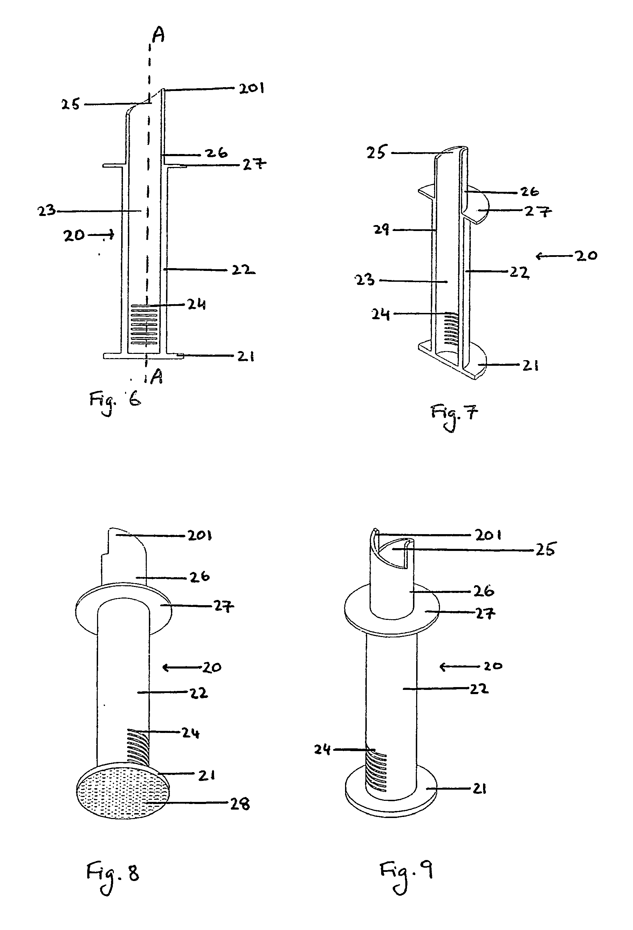

Figures 6 to 9 show a grinder (20) of the present invention. The grinder (20) has a grinding head (21), a cylindrical shaft (22), a central axis (A) and a protruding top or portion (26). The grinder (20) is preferably constructed as a single unit, but this is a matter of manufacturing inclination.

The grinding head (21) is a solid element, which is formed to be receivable within the interior bottom (5) of the container (1) (see Figures 2 and 5). The grinding head (21) is shaped to substantially conform to the shape of the interior bottom (5), having a substantially flat disc shape similar to that of the interior bottom (5) and adapted to be received therein in a generally surface-to-surface relation.

In alternative embodiments of the invention, the shape of the grinding head (21) is shaped so as to accommodate the interior bottom (5), for example, the respective shapes may be selected from conical, frustoconical, spherical cap shaped, hemispherical shaped and so on.

The surface of the grinding head (21) proximate to the interior bottom (5) preferably comprises a sample-engaging surface in the form of a grinding abrasive surface (28). The grinding abrasive surface (28) is integrally formed with the surface of the grinding head (21) and is composed of many protrusions on the surface of the grinding head (21). It will be appreciated that alternative embodiments of the present invention may function without an abrasive surface, and also that the grinding abrasive surface (28) may be generated by other means, such as hatching or scoring the surfaces, or the addition of griddle lines or similar protrusions. Alternatively the grinding abrasive surface (28) may comprise a particulate substance wherein the average particle size is between about 36 µm and about 1800 µm and preferably the average particle size is about 425 µm.

The perimeter (29) of the shaft (22) of the grinder (20) defines an interior channel (23), wherein the grinding head (21) defines the closed base of the shaft. The shaft (22) of the present embodiment is cylindrical, but it will be appreciated that this is not an essential feature of the invention. The shaft (22) has an upper or protruding portion (26), which defines an upwardly open mouth or top (25) to the grinder (20). The open mourn (25) is distal to the grinding head (21).

Referring to figures 6 to 9, at the end of the grinder (20) proximate to the grinding head (21) of the shaft (22) is located a series of ports (24). The ports (24) are located on the perimeter (29) of the shaft proximate to the grinding head (21). The ports (24) are dimensioned so that homogenised or comminuted material can pass into the interior channel (23) of the shaft (22) while non homogenised or comminuted material remains in the interior (2) of the container (1). Thus, when in use the apparatus may communicate homogenised or comminuted material from the interior (2) of the container (1) through the ports (24) and the interior (23) and out the open mouth (25) of the shaft (22). In one embodiment the homogenised or comminuted material is removed by aspiration.

While the present embodiment is depicted with a series of ports (24), it will be appreciated that other embodiments may have a single port, or a plurality of ports of various shapes in various arrangements about the shaft (22). Furthermore, in an alternative embodiment of the present invention, the ports can be reversibly closed, for example, by means of an inner cylindrical shaft which can notate or elevate within the shaft (22). The ports can be arranged to suit the needs of the end use, for example, the port(s) can be located at a defined level of the vertical length of the shaft (22) in order to facilitate the removal of particular fractions of the comminuted material, for example, those fractions contained in lipid, organic or phenol based fractions relative to aqueous fractions and so on.

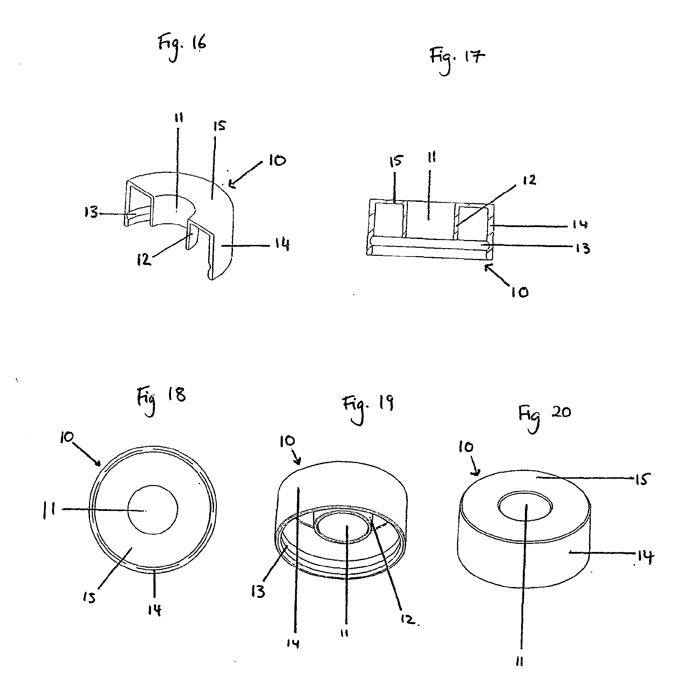

Figures 16 to 20 shows a cap (10) that is engagable with the upper portion of the container (4). The cap comprises a generally tubular body (33), open at one end, having an outer wall or skirt (14) connected to a shaft engaging means, in the form of a co-axial internal continuous sidewall (12) which is spaced radially inward from the outer wall or skirt (14) and connected thereto at or adjacent to the other end of the body by a roof portion (15). It will be appreciated by those skilled in the art that the presence of the internal continuous sidewall (12) is not necessary for the functioning of the apparatus, but does provide a stabilising action against lateral movement of the shaft (22).

The internal continuous sidewall (12) is of reduced length relative to the outer wall or skirt (14). The internal continuous sidewall (12) defines an aperture (11). The side of the internal continuous sidewall (12) proximate to the aperture (11) may be coated with and/or comprise a low friction surface that when engaged with the grinder (20) simultaneously both provides a close-fitting seal between the cylindrical shaft (22) of the grinder (20), and reduces friction when the cylindrical shaft (22) of the grinder (20) is rotated at speed (see Figures 2 and 5).

In the embodiment shown in the figures 2 and 5, and the cap (10) and the container (1) mate using a push-fit and snap-lock arrangement. The snap-lock arrangement involves an annular recess (13) located on the internal face of the outer wall or skirt (14) of the cap (10) detachably mating with a complementary annular rim (9) located on the exterior face of the portion of the container (1) distal to the interior bottom (5).

In an alternative embodiment the cap comprises a neck with at least one screw thread or annular rim or recess wherein the cap (10) and the container (1) mate through a thread and screw arrangement.

Referring to figures 1 to 5, a preferred embodiment of the invention is depicted wherein the circumference of the protruding portion of the shaft (22) is slightly less than the circumference of the aperture (11) of the cap (10) so that the grinder (20) is closely received by the aperture (11) of the cap (10). The cylindrical shaft (22) further comprises a restraining means in the form of disc-shaped collar (27). Referring to Figure 2 and 5, when the cap (10) is engaged with the container (1), with the grinder (20) *in situ,* the collar (27) engages with the internal continuous sidewall (12) and limits the amount of the shaft that can emerge from the aperture (11). The collar (27) has the additional advantage of limiting the egress of aerosols and debris through the aperture during homogenisation.

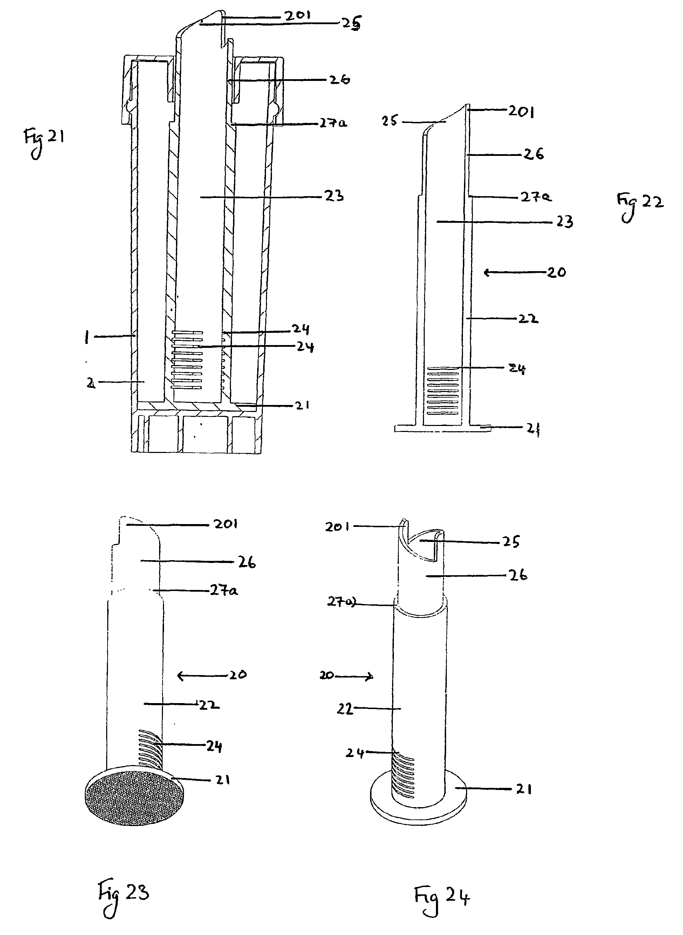

Figures 21 to 24 depict an alternative embodiment where the restraining means takes the form of a shoulder (27a) so that the radius of the protruding portion (26) of the shaft (22) is less than the radius of the shaft (22). In this embodiment the annular rim (27a) of the top or protruding portion (26) of the grinder (20) is adapted to be closely received by that portion of the internal continuous sidewall (12) most distal to the annular end wall portion (15) of the cap (10).

Referring to figures 1 to 5 and 21 to 25, when the grinder (20) is engaged with the cap (10) an engagement means (201) of the grinder (20) is accessible to the exterior of the apparatus. The protruding portion of the grinder (26) may protrude beyond the interior (2) of the container (1). In one embodiment the engagement means (201) is dimensioned to form two 180° helices in which the end of each helix most distal to the grinding head (21) is separated from the other by 180° of the circumference of the cylindrical shaft (22). It is preferable that the uppermost tips of each helix are blunted, as depicted in figures 6 to 9. This prevents the grinder (20) from presenting a sharp point, which could present a risk of injury to the user as well as potentially damaging storage or transport packaging of the comminuter.

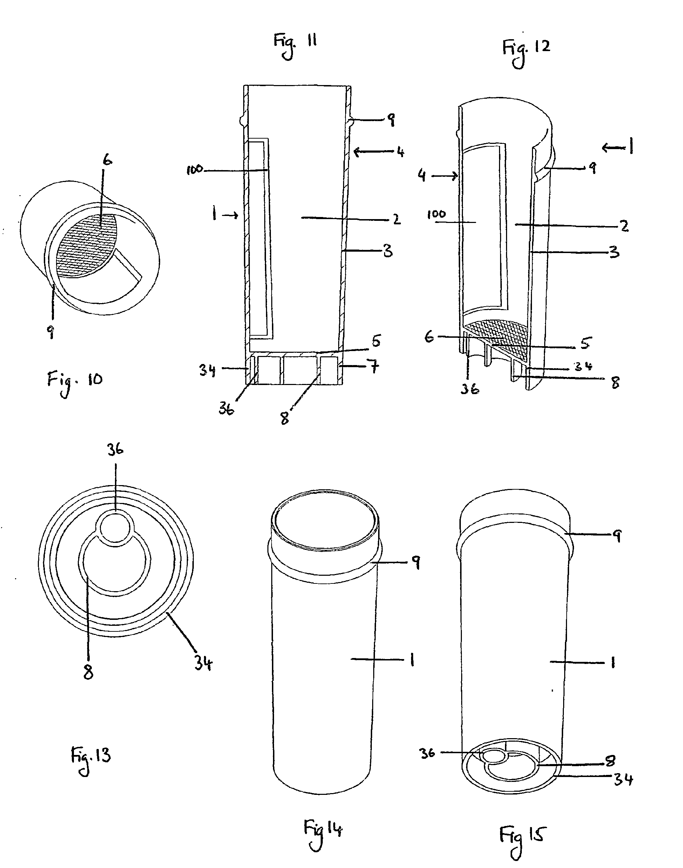

The container (1) will now be described with reference to figures 10 to 15 wherein the container (1) has an interior (2) defined with sidewalls (3), an upper portion (4) having an upwardly open top (31), and an interior bottom surface (5) The container (1) is preferably constructed of clear plastics material such as polypropylene so that an operator can ascertain by way of visual inspection that the sample has been satisfactorily homogenised. In particular the sidewalls (3) preferably have a polished finish on both the interior and exterior of the walls.

The interior bottom surface (5) is formed with a sample-engaging surface, in the form of a receiving abrasive surface (6). The receiving abrasive surface (6) is integrally formed with the interior bottom surface (5) and is composed of many protrusions on the interior bottom surface (5). It will be appreciated that alternative embodiments of the present invention may function without a receiving abrasive surface (6), and also that the receiving abrasive surface (6) may be generated by other means, such as hatching or scoring the surface, or the addition of griddle lines or similar indentations or protrusions.

The interior bottom (5) is preferably substantially perpendicular to the central axis (32) of the container (1) presenting a flat surface to a grinding head (21) of a grinder (20). In alternative embodiments of the invention, the interior bottom (5) is shaped so as to accommodate the shape of the grinding head (21), for example, conical shaped, frustoconical shaped, spherical cap shaped, hemispherical shaped and so on.

In some embodiments an area (100) on the exterior sidewall (3) may be provided with a spark-eroded finish to facilitate the printing of a unique marking to aid in the identification of the container and thus the containers contents. Such markings are suitably barcodes, global unique identifier (GUID) or the like.

The base of the container (1) is defined by the circular continuous lower sidewall (34) that defines the outer circumference of the base. The interior bottom (5) serves to divide the interior (2) from a base attachment portion (7) of the container (1).

The circular continuous lower sidewall (34) is connected to an inner sidewall or skirt (8) by the interior bottom (5). The inner sidewall (8) is spaced radially inward from the circular continuous sidewall (3). The inner sidewall (8) is approximately concentric with an axis of the container. In an alternative embodiment, a guiding means in the form of a notch can be provided on the inner sidewall (8). The inner sidewall (8) is also formed in contact with a securing means, in the form of a guiding ring (36). The guiding ring (36) is a circular wall superimposably intersecting the circumference of the inner sidewall (8) such that the centre of the circle defining the guiding ring (36) is distinct and separate from the central axis (32).

In alternative embodiments of the invention, the inner sidewall (8) is of reduced length relative to the circular continuous sidewall (3). In a further embodiment the inner sidewall (8) comprises an internal sidewall connecting two points within the ellipse.

The provision at the base of the container (1) of the inner sidewall (8), and guiding ring (36) in conjunction with the circular continuous sidewall (3) provides a means by which the apparatus may be stably mounted within a holding device during the homogenisation process. This also permits the container to be located in a fixed position relative to a device such as bar code scanner or the like.

Preferably, the apparatus and/or its constituent parts are injection moulded from plastics, for example thermoplastics, but will be appreciated that a wide variety of other materials are suitable, for example, metals, ceramics and glass.

In general, it may not be preferable for the apparatus to be manufactured from polystyrene, as certain biological tissues and moieties are known to stick to it. However, it will be understood that in certain applications, certain embodiments of the invention may have portions of the apparatus composed or coated with a material specifically designed to adhere to or react with certain biological material, for example antibodies or enzymes and the like. Furthermore, the coating could be applied to any portion of the apparatus likely to come in contact with the sample or comminuted material.

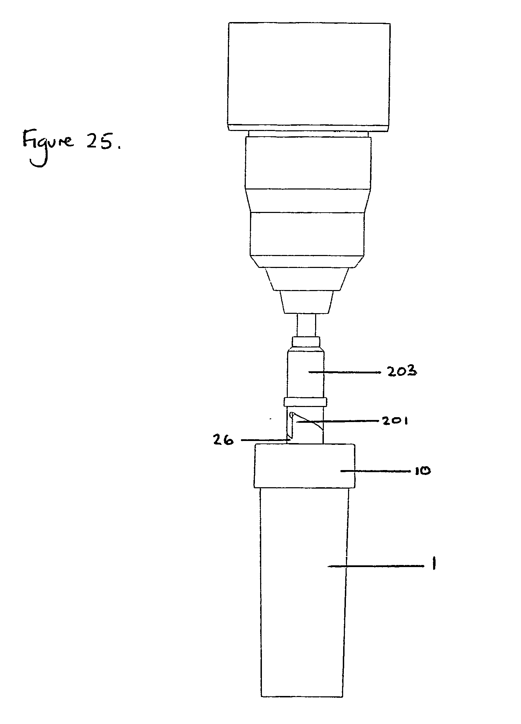

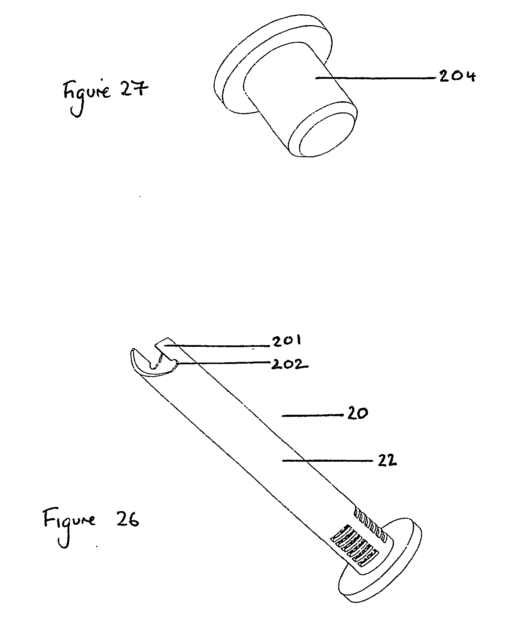

Figure 26 depicts a further embodiment of the invention, where the engaging means (201) also comprises a coupling means (202), to assist the coupling of the grinder (20) to either the rotation means (203) or a closure means, such as a bung (204) as illustrated in figure 27. A rotation means (203) may detachably engage with each embodiment of the engagement means (201) of the grinder (20) (Figure 25). The rotation means (201) can be mechanically driven or operated manually

The combination of the downward and rotary forces applied to the grinding head (21) serves to grind and/or comminute tissue specimen between the grinding head (21) and the interior bottom (5) of the container

A biasing means may be incorporated into, or with, the cylindrical shaft (22) and/or cap (10) so that the grinding head (21) of the grinder (20) is placed at a distance from the receiving abrasive surface (6) of the interior bottom (5) in a consistent manner so that during homogenisation of samples each use of the apparatus reliably produces the same degree of homogenisation.

In use, the apparatus is placed in what can be described as a "grinding configuration" that is, a sample of tissue, for example central nervous system tissue, is deposited in the interior (2) of the container (1) on the interior bottom (5). The grinder (20) is then placed within the interior (2) of the container (1) with the grinding head (20) received in the interior bottom (5) and spaced therefrom by the tissue sample. The cap (20) is then placed on the sidewalls (3) of the container distal to the interior bottom (5). The cap (10) is engaged with the container (1) through a fastening mechanism such as a snap lock or screw-thread-type arrangement.

The apparatus can then be mounted or engaged with a rotating means, wherein the base attachment portion (7) of the container and the protruding portion (26) of the grinder are engaged with support and rotating means respectively.

With the apparatus thus placed in the grinding configuration, the sample may be homogenised or comminuted into a substantially liquid form, so that a significant proportion of the sample may pass through the ports (24) into the interior channel (23) wherein subsequent aspiration of the sample from the apparatus may occur.

Where the rotation means is mechanically powered, the speed of revolution of the shaft (22) can produce a centrifugal force sufficient to prevent liquid entering the interior channel (23) of the grinder (20) through the ports (24).

It is appreciated that certain features of the invention, which are, for clarity, described in the context of separate embodiments, may also be provided in combination in a single embodiment. Conversely, various features of the invention that are, for brevity, described in the context of a single embodiment, may also be provided separately or in any suitable sub-combination within the scope defined by the claims.

The words "comprises/comprising" and the words "having/including" when used herein with reference to the present invention are used to specify the presence of stated features, integers, steps or components but does not preclude the presence or addition of one or more other features, integers, steps, components or groups thereof.

## Claims

1. A homogeniser for comminuting a sample of material comprising a grinder (20), wherein the grinder (20) comprises
(i) a hollow shaft (22) defining an interior channel (23),
(ii) a grinding head (21) and
(iii) at least one port (24);
**characterized in that** the grinding head defines a closed end of the shaft (22) and the at least one port (24) is located on the elongate sides of the shaft (22), said port being dimensioned so that only comminuted material may flow into the interior channel (23), of the shaft (22).

2. A homogeniser as claimed in claim 1, wherein the port (24) comprises a slit formed in the shaft (22).

3. A homogeniser as claimed in any preceding claim wherein the port (24) is positioned on the shaft (22) proximate to the grinding head (21).

4. A homogeniser as claimed in any preceding claim, wherein there is provided a means to reversibly close the at least one port (24).

5. A homogeniser as claimed in any preceding claim, wherein the at least one port (24) is positioned on the shaft (22) such that when the homogeniser is used in combination with a defined volume of a solution separable into at least two layers defined by differing density gradients, the positioning of the at least one port permits egress of at least one of the layers into the interior channel (23) and permits the prevention of egress of at least one of the layers into the interior channel (23).

6. A homogeniser as claimed in any preceding claim, wherein the upper portion of the shaft (26) is provided with an engagement means (201), the engagement means (201) providing a means of detachably engaging the shaft (22) with a rotation device (203), or a rotation device adaptor, or a closure means (204).

7. A homogeniser as claimed in any of claims 1-6, further comprising a means to aspirate the homogenate from the inner channel (23).

8. A homogeniser as claimed in any preceding claim, wherein the grinding head (21) comprises at least one blade.

9. A homogeniser as claims in any preceding claim, further comprising a container (1), the container comprising an interior (2) defined by sidewalls (3), an upper portion (4) having an upwardly open top communicable with an exterior of the container, and an interior bottom (5), such that the hollow shaft (22) is locatable within the container.

10. A homogeniser as claimed in claim 9, wherein the grinding head (21) is shaped to compliment the shape of the interior bottom (5), preferably wherein the shape is selected from the group consisting of substantially flat, substantially conical, substantially frustroconical, substantially hemispherical and substantially spherical-cap shaped.

11. A homogeniser as claimed in any one of claims 9-10, wherein the interior bottom (5) and the grinding head (21) each comprise a sample-engaging surface.

12. A homogeniser as claimed in claim 11, wherein at least one of the sample-engaging surfaces is an abrasive surface, preferably wherein the abrasive surface comprises at least one abrasive feature selected from the group consisting of protrusions, griddles, indentations, hatching or embedded particles.

13. A homogeniser as claimed in any one of claims 9-12, wherein the shaft (22) further comprises a biasing means for biasing the grinding head (21) against the interior bottom (5) of the container (1).

14. A homogeniser as claimed in any one of claims 9-13, wherein the grinder (20) can nest within the container (1), such that the grinding head (21) is proximate to the interior bottom (5), and a sample of comminutable material can be contained between the grinding head (21) and the interior bottom (5), and movement of the grinder (20) relative to the container results in the sample of material being comminuted.

15. A homogeniser as claimed in claim 14, wherein the grinder (20) is moveable in at least one direction chosen from rotational movement and movement in a direction substantially parallel to the central axis (A).

16. A homogeniser as claimed in any one of claims 9 - 15, further comprising a cap (10), wherein the cap (10) comprises a roof portion (15) and an outer skirt (14) engagable with the upper portion of the container (4) and the roof portion (15) further comprises an aperture (11) adapted to permit the shaft (22) of the grinder (20) to protrude through the aperture (11).

17. A homogeniser as claimed in claim 16, further comprising a shaft engaging means and a portion of the shaft (22) is dimensioned to be engagable with the shaft engaging means.

18. A homogeniser as claimed in any of claims 16-17, wherein the shaft (22) is provided with a restraining means to limit the movement of the shaft (22) through the aperture (11).

19. A homogeniser as claimed in any one of claims 9-18, further comprising a holding device.

20. A homogeniser as claimed in claim 19, wherein the container further comprises a positioning means engagable with the holding device, such that the positioning means permits the container (1) to be engaged in a specific orientation relative to the holding device.

21. A homogeniser as claimed in any one of claims 9-20, further comprising a rotation device.

22. A homogeniser as claimed in any preceding claim wherein at least one portion of the homogeniser is composed of, impregnated with, or coated with a reactive material selected to react with a moiety intended for use within the homogeniser.

23. A kit for homogenising a sample of material comprising at least one homogeniser as claimed in any preceding claim and optionally further comprising at least one of the group consisting of a rotation device, a rotation device adapter, a closure means or a holding device.

24. A method for homogenising a sample of material comprising the use of a homogeniser as claimed in any of claims 9 to 22, and
(i) placing the sample to be homogenised within the interior (2) of the container (1) such that it can be retained by the interior bottom (5),
(ii) inserting the shaft (20) into the interior (2) such that the grinding head is contactable with the sample,
(iii) placing the cap (10) on the container (1) such that the upper portion (26) of the shaft (22) extends through the aperture (11),
(iv) engaging the engagement means (201) with the rotation device and optionally securably restraining the rotational movement of the container (1) relative to the shaft (22) optionally by means of the holding device.
(v) homogenising the sample material by means of movement of the shaft (22) relative to the container (1),
(vi) removing homogenised material from the interior of the container (1) into the interior channel (23) of the shaft (22) by means of at least one port (24).

## Patentansprüche

1. Homogenisator zum Zerkleinern einer Materialprobe, mit einem Mahlwerk (20), wobei das Mahlwerk (20) aufweist:
(i) eine hohle Welle (22), in der ein Innenkanal (23) ausgebildet ist,
(ii) einen Mahlkopf (21) und
(iii) mindestens einen Durchlass (24);
**dadurch gekennzeichnet, dass** der Mahlkopf ein geschlossenes Ende der Welle (22) bildet und dass der mindestens eine Durchlass (24) an den Längsseiten der Welle (22) angeordnet ist, wobei der Durchlass derart bemessen ist, dass nur zerkleinertes Material in den Innenkanal (23) der Welle (22) strömen kann.

2. Homogenisator nach Anspruch 1, bei dem der Durchlass (24) einen in der Welle (22) ausgebildeten Schlitz aufweist.

3. Homogenisator nach einem der vorhergehenden Ansprüche, bei dem der Durchlass (24) nahe dem Mahlkopf (21) an der Welle (22) angeordnet ist.

4. Homogenisator nach einem der vorhergehenden Ansprüche, bei dem eine Vorrichtung zum reversiblen Schließen des mindestens einen Durchlasses (24) vorgesehen ist.

5. Homogenisator nach einem der vorhergehenden Ansprüche, bei dem der mindestens eine Durchlass (24) derart an der Welle (22) positioniert ist, dass, wenn der Homogenisator in Kombination mit einem definierten Volumen einer Lösung verwendet wird, die in mindestens zwei durch verschiedene Dichtegradienten definierte Lagen separierbar ist, die Positionierung des mindestens einen Durchlasses ein Austreten mindestens einer der Lagen in den Innenkanal (23) zulässt und ein Austreten mindestens einer der Lagen in den Innenkanal (23) verhindert.

6. Homogenisator nach einem der vorhergehenden Ansprüche, bei dem der obere Teil (26) der Welle mit einer Ankopplungsvorrichtung (201) versehen ist, wobei die Ankopplungsvorrichtung (201) eine Vorrichtung zur lösbaren Kopplung der Welle (22) mit einer Drehvorrichtung (203) oder einem Drehvorrichtungsadapter oder einer Schließvorrichtung (204) bildet.

7. Homogenisator nach einem Ansprüche 1-6, ferner mit einer Vorrichtung zum Ansaugen des Homogenats aus dem Innenkanal (23).

8. Homogenisator nach einem der vorhergehenden Ansprüche, bei dem der Mahlkopf (21) mindestens eine Klinge aufweist.

9. Homogenisator nach einem der vorhergehenden Ansprüche, ferner mit einem Behälter (1), wobei der Behälter einen Innenraum (2) aufweist, der begrenzt ist durch Seitenwände (3), ein Oberteil (4) mit einem nach oben offenen Ende, das mit dem außerhalb des Behälters gelegenen Bereich verbindbar ist, und einen inneren Boden (5), derart, dass die hohle Welle (22) in dem Behälter platzierbar ist.

10. Homogenisator nach Anspruch 9, bei dem der Mahlkopf (21) eine der Form des inneren Bodens (5) komplementäre Form hat, die vorzugsweise aus der Gruppe gewählt ist, zu der eine im Wesentlichen flache, eine im Wesentlichen konische, eine im Wesentlichen kegelstumpfartige, eine im Wesentlichen halbkugelartige und eine im Wesentlichen kugelkappenartige Form zählen.

11. Homogenisator nach einem Ansprüche 9-10, bei dem der inneren Boden (5) und der Mahlkopf (21) jeweils eine Probenangriffsfläche aufweisen.

12. Homogenisator nach Anspruch 11, bei dem mindestens eine der Probenangriffsflächen eine Abrasivfläche ist, wobei die Abrasivfläche vorzugsweise mindestens ein Abrasivmerkmal aufweist, das aus der Gruppe gewählt ist, zu der Vorsprünge, Rippenstrukturen, Vertiefungen, Schraffierungen oder eingebettete Partikel zählen.

13. Homogenisator nach einem Ansprüche 9-12, bei dem die Welle (22) ferner eine Vorspannvorrichtung zum Vorspannen des Mahlkopfs (21) gegen den inneren Boden (5) des Behälters (1) aufweist.

14. Homogenisator nach einem Ansprüche 9-13, bei dem das Mahlwerk (20) derart in dem Behälter (1) eingebettet sein kann, dass der Mahlkopf (21) nahe dem inneren Boden (5) angeordnet ist, eine Probe zerkleinerbaren Materials zwischen dem Mahlkopf (21) und dem inneren Boden (5) eingeschlossen sein kann und eine Bewegung des Mahlwerks (20) relativ zu dem Behälter dazu führt, dass die Materialprobe zerkleinert wird.

15. Homogenisator nach Anspruch 14, bei dem das Mahlwerk (20) in mindestens einer Richtung bewegbar ist, die gewählt ist aus einer Drehbewegungsrichtung und einer im Wesentlichen parallel zu der Mittelachse (A) verlaufenden Bewegungsrichtung.

16. Homogenisator nach einem Ansprüche 9-15, ferner mit einem Deckel (10), wobei der Deckel (10) einen Dachteil (15) und eine Außenschürze (14) aufweist, die mit dem Oberteil (4) des Behälters in Eingriff bringbar ist, und wobei der Dachteil (15) ferner eine Öffnung (11) aufweist, welche die Welle (22) des Mahlwerks (20) hindurchtreten lässt.

17. Homogenisator nach Anspruch 16, bei dem ferner eine Wellenankopplungsvorrichtung vorgesehen ist und ein Teil der Welle (22) derart dimensioniert ist, dass er mit der Wellenankopplungsvorrichtung koppelbar ist.

18. Homogenisator nach einem Ansprüche 16-17, bei dem die Welle (22) mit einer Rückhaltevorrichtung versehen ist, um die Bewegung der Welle (22) durch die Öffnung (11) zu beschränken.

19. Homogenisator nach einem Ansprüche 9-18, ferner mit einer Haltevorrichtung.

20. Homogenisator nach Anspruch 19, bei dem der Behälter ferner eine Positionierungsvorrichtung aufweist, die mit der Haltevorrichtung derart in Eingriff bringbar ist, dass die Positionierungsvorrichtung ein Verrasten des Behälters (1) in einer bestimmten Ausrichtung relativ zu der Haltevorrichtung ermöglicht.

21. Homogenisator nach einem Ansprüche 9-20, ferner mit einer Drehvorrichtung.

22. Homogenisator nach einem der vorhergehenden Ansprüche, wobei mindestens ein Teil des Homogenisators aus einem reaktiven Material besteht oder mit diesem imprägniert oder beschichtet ist, das dahingehend gewählt ist, dass es mit einer zur Verwendung in dem Homogenisator vorgesehenen Struktureinheit reagiert.

23. Ausrüstungssatz zum Homogenisieren einer Materialprobe, mit mindestens einem Homogenisator nach einem der vorhergehenden Ansprüche und optional mit mindestens einer Vorrichtung aus der Gruppe, zu der eine Drehvorrichtung, ein Drehvorrichtungsadapter, eine Verschlussvorrichtung und eine Haltevorrichtung zählen.

24. Verfahren zum Homogenisieren einer Materialprobe unter Verwendung eines Homogenisator nach einem der Ansprüche 9 bis 22 und mit folgenden Schritten:
(i) Platzieren der zu homogenisierenden Probe in dem Inneren (2) des Behälters (1) derart, dass sie von dem inneren Boden (5) gehalten werden kann,
(ii) Einführen der Welle (20) in das Innere (2) derart, dass der Mahlkopf in Kontakt mit der Probe bringbar ist,
(iii) Platzieren des Deckels (10) an dem Behälter (1) derart, dass sich der obere Teil (26) der Welle (22) durch die Öffnung (11) erstreckt,
(iv) Ankoppeln der Ankopplungsvorrichtung (201) an die Drehvorrichtung und, optional, sicherbares Beschränken der Drehbewegung des Behälters (1) relativ zu der Welle (22), und zwar optional mittels der Haltevorrichtung,
(v) Homogenisierung des Probenmaterials mittels Bewegung der Welle (22) relativ zu dem Behälter (1),
(vi) Entnehmen homogenisierten Materials aus dem Inneren des Behälters (1) heraus in den Innenkanal (23) der Welle (22) durch mindestens einen Durchlass (24).

## Revendications

1. Homogénéisateur pour concasser un échantillon de matériau, comprenant une meule (20), dans lequel la meule (20) comprend :
(i) un arbre creux (22) définissant un canal intérieur (23),
(ii) une tête de meulage (21), et
(iii) au moins un orifice (24) ;
**caractérisé en ce que** la tête de meulage définit une extrémité fermée de l'arbre (22) et ledit au moins un orifice (24) est positionné sur les côtés allongés de l'arbre (22) ledit orifice étant dimensionné de sorte que seul le matériau concassé peut s'écouler dans le canal intérieur (23) de l'arbre (22).

2. Homogénéisateur selon la revendication 1, dans lequel l'orifice (24) comprend une fente formée dans l'arbre (22).

3. Homogénéisateur selon l'une quelconque des revendications précédentes, dans lequel l'orifice (24) est positionné sur l'arbre (22) à proximité de la tête de meulage (21).

4. Homogénéisateur selon l'une quelconque des revendications précédentes, dans lequel, on prévoit des moyens pour fermer de manière réversible ledit au moins un orifice (24).

5. Homogénéisateur selon l'une quelconque des revendications précédentes, dans lequel au moins un orifice (24) est positionné sur l'arbre (22) de sorte que lorsque l'homogénéisateur est utilisé en combinaison avec un volume défini d'une solution séparable en au moins deux couches définies par des gradients de densité différents, le positionnement dudit au moins un orifice permet la sortie d'au moins l'une des couches dans le canal intérieur (23) et permet d'empêcher la sortie d'au moins l'une des couches dans le canal intérieur (23).

6. Homogénéisateur selon l'une quelconque des revendications précédentes, dans lequel la partie supérieure de l'arbre (26) est dotée de moyens de mise en prise (201), les moyens de mise en prise (201) fournissant des moyens pour mettre en prise de manière détachable l'arbre (22) avec un dispositif de rotation (203), ou un adapteur de dispositif de rotation ou des moyens de fermeture (204).

7. Homogénéisateur selon l'une quelconque des revendications 1 à 6, comprenant en outre les moyens pour aspirer le broyat du canal interne (23).

8. Homogénéisateur selon l'une quelconque des revendications précédentes, dans lequel la tête de meulage (21) comprend au moins une pale.

9. Homogénéisateur selon l'une quelconque des revendications précédentes, comprenant en outre un récipient (1), le récipient comprenant un intérieur (2) défini par des parois latérales (3), une partie supérieure (4) ayant une partie supérieure ouverte vers le haut pouvant communiquer avec un extérieur du récipient, et un fond intérieur (5), de sorte que l'arbre creux (22) peut être positionné à l'intérieur du récipient.

10. Homogénéisateur selon la revendication 9, dans lequel la tête de meulage (21) est formée pour se conformer à la forme du fond intérieur (5), de préférence dans lequel la forme est choisie dans le groupe comprenant des formes sensiblement plates, sensiblement coniques, sensiblement tronconiques, sensiblement hémisphériques et sensiblement en forme de capuchons sphériques.

11. Homogénéisateur selon l'une quelconque des revendications 9-10, dans lequel le fond intérieur (5) et la tête de meulage (21) comprennent chacun une surface de mise en prise d'échantillon.

12. Homogénéisateur selon la revendication 11, dans lequel au moins l'une des surfaces de mise en prise d'échantillon est une surface abrasive, de préférence dans lequel la surface abrasive comprend au moins une caractéristique abrasive choisie dans le groupe comprenant les saillies, les tamis, les dentelures, les hachures ou les particules encastrées.

13. Homogénéisateur selon l'une quelconque des revendications 9 à 12, dans lequel l'arbre (22) comprend en outre des moyens de sollicitation pour solliciter la tête de meulage (21) contre le fond intérieur (5) du récipient (1).

14. Homogénéisateur selon l'une quelconque des revendications 9 à 13, dans lequel la meule (20) peut s'emboîter à l'intérieur du récipient (1), de sorte que la tête de meulage (21) est à proximité du fond intérieur (5), et un échantillon de matériau concassable peut être contenu à l'intérieur de la tête de meulage (21) et le fond intérieur (5), et le mouvement de la meule (20) par rapport au récipient se traduit par l'échantillon du matériau qui est concassé.

15. Homogénéisateur selon la revendication 14, dans lequel la meule (20) est mobile dans au moins une direction choisie à partir du mouvement de rotation et du mouvement dans une direction sensiblement parallèle à l'axe central (A).

16. Homogénéisateur selon l'une quelconque des revendications 9 à 15, comprenant en outre un capuchon (10), dans lequel le capuchon (10) comprend une partie de toit (15) et une jupe externe (14) pouvant se mettre en prise avec la partie supérieure du récipient (4) et la partie de toit (15) comprend en outre une ouverture (11) adaptée pour permettre à l'arbre (22) de la meule (20) de faire saillie à travers l'ouverture (11).

17. Homogénéisateur selon la revendication 16, comprenant en outre des moyens de mise en prise d'arbre et une partie de l'arbre (22) est dimensionnée pour pouvoir se mettre en prise avec les moyens de mise en prise d'arbre.

18. Homogénéisateur selon l'une quelconque des revendications 16-17, dans lequel l'arbre (22) est prévu avec des moyens de limitation pour limiter le mouvement de l'arbre (22) à travers l'ouverture (11).

19. Homogénéisateur selon l'une quelconque des revendications 9 à 18, comprenant en outre un dispositif de support.

20. Homogénéisateur selon la revendication 19, dans lequel le récipient comprend en outre des moyens de positionnement pouvant être mis en prise avec le dispositif de support, de sorte que les moyens de positionnement permettent au récipient (1) d'être mis en prise dans une orientation spécifique par rapport au dispositif de support.

21. Homogénéisateur selon l'une quelconque des revendications 9 à 20, comprenant en outre un dispositif de rotation.

22. Homogénéisateur selon l'une quelconque des revendications précédentes, dans lequel au moins une partie de l'homogénéisateur est composée de, imprégnée avec ou recouverte avec un matériau réactif choisi pour réagir avec un fragment prévu pour être utilisé à l'intérieur de l'homogénéisateur.

23. Trousse pour homogénéiser un échantillon de matériau comprenant au moins un homogénéisateur selon l'une quelconque des revendications précédentes et comprenant en outre facultativement au moins le groupe se composant d'un dispositif de rotation, d'un adaptateur de dispositif de rotation, de moyens de fermeture ou d'un dispositif de support.

24. Procédé pour homogénéiser un échantillon de matériau comprenant l'utilisation d'un homogénéisateur selon l'une quelconque des revendications 9 à 22, et les étapes consistant à :
(i) placer l'échantillon à homogénéiser à l'intérieur (2) du récipient (1) de sorte qu'il peut être retenu par le fond intérieur (5) ;
(ii) insérer l'arbre (20) dans l'intérieur (2) de sorte que la tête de meulage peut être en contact avec l'échantillon ;
(iii) placer le capuchon (10) sur le récipient (1) de sorte que la partie supérieure (26) de l'arbre (22) s'étend à travers l'ouverture (11),
(iv) mettre en prise les moyens de mise en prise (201) avec le dispositif de rotation et limiter facultativement de manière sûre le mouvement de rotation du récipient (1) par rapport à l'arbre (22) facultativement au moyen du dispositif de support,
(v) homogénéiser le matériau d'échantillon au moyen du mouvement de l'arbre (22) par rapport au récipient (1),
(vi) retirer le matériau homogénéisé de l'intérieur du récipient (1) dans le canal intérieur (23) de l'arbre (22) au moyen d'au moins un orifice (24).
